## Europäisches Patentamt

## European Patent Office

## Office européen des brevets

(11) Publication number: **0 052 404**
**B1**

## EUROPEAN PATENT SPECIFICATION

(45) Date of publication of patent specification: **27.05.87**

(51) Int. Cl.⁴: **A 61 K 9/08**

(21) Application number: **81201252.4**

(22) Date of filing: **09.11.81**

(54) Non-yellowing topical pharmaceutical composition.

(30) Priority: **19.11.80 US 208445**

(43) Date of publication of application:
**26.05.82 Bulletin 82/21**

(45) Publication of the grant of the patent:
**27.05.87 Bulletin 87/22**

(84) Designated Contracting States:
**BE CH DE FR GB IT LI LU NL SE**

(56) References cited:
**FR-A-2 204 402**
**US-A-2 988 569**
**US-A-3 527 864**
**US-A-3 952 099**

(73) Proprietor: **THE PROCTER & GAMBLE COMPANY**
**301 East Sixth Street**
**Cincinnati Ohio 45202 (US)**

(72) Inventor: **Koonsvitsky, Burton Phillip**
**4302 Berryhill Lane**
**Cincinnati Ohio 45242 (US)**
Inventor: **Manring, Gary Lee**
**5575 Stillwell Road**
**Oxford Ohio 45056 (US)**

(74) Representative: **Suslic, Lydia et al**
**Procter & Gamble European Technical Center**
**N.V. Temselaan 100**
**B-1820 Strombeek-Bever (BE)**

Courier Press, Leamington Spa, England.

## Description

The invention described and claimed herein relates to pharmaceutical compositions formulated for topical application, containing tetracycline, tetracycline salts or tetracycline derivatives, used in the treatment of a variety of skin disorders.

The use of antibiotics, especially tetracycline, is a widely accepted mode of therapy for a variety of conditions, including skin disorders, such as acne. Especially in the case of skin disorders, the convenience and the fact that application of the active ingredient is made directly to the afflicted situs with only a minimal systemic exposure to the active component, makes topical application a preferred mode of administration. However, the topical use of tetracycline or tetracycline-derived compounds, can lead to a yellowing of the skin at the site of application, particularly in people with fair complexions and pink skin tones. Although this skin yellowing is temporary and can be washed away by normal soap and water washing, it can be viewed by users of the topical tetracycline composition as a cosmetically undesirable characteristic and can even be perceived in efficacy terms since, as a result of the skin yellowing, the patient may decrease usage of the tetracycline composition, causing an exacerbation of the condition being treated. Thus, it would be very desirable from both cosmetic and treatment viewpoints, to be able to formulate a tetracycline-containing topical composition which does not exhibit this skin yellowing characteristic.

The use of topical pharmaceutical compositions, some of which contain tetracycline, in the treatment of a wide variety of conditions, such as acne, is known in the art. See, for example, US—A—3,896,238 and US—A—3,527,864. Citric acid is known for use as a component in a variety of therapeutic treatments, such as in the preparation of effervescent granules, the enhancement of antioxidant effectiveness, as a disinfectant, and in a lotion for the neutralization of alkali materials in the eyes. See, Martindale, *The Extra Pharmacopoeia*, 26th Edition, The Pharmaceutical Press, 1972, pages 880—881. In addition, citric acid has been used at low levels (i.e., less than about 0.1%) as a stabilizer in various pharmaceutical products. US—A—2,988,569 describes a procedure utilizing a pH of less than 2, for producing stable impurity-free tetracycline antibiotics.

It has now been found that by carefully controlling the size and configuration of tetracycline crystals as they recrystallize at the skin surface after application, it is possible to formulate topical tetracycline-containing compositions which do not yellow the skin upon use. This is accomplished by buffering the compositions at a pH between 2.0 and 4, preferably using citric acid as the buffering acid.

It is, therefore, an object of the present invention to provide topical pharmaceutical compositions, containing tetracycline or tetracycline-derived compounds, which do not yellow the skin upon use.

The present invention provides topical compositions for use in the treatment of skin disorders, which comprise: (1) from 0.005% to 5% of a tetracycline compound selected from tetracycline, a tetracycline salt or a tetracycline derivative, in solution; (2) from 0.5% to 10% of a pharmaceutically-acceptable acid which buffers the composition at a pH of from 2.0 to 4; and (3) a pharmaceutically-acceptable topical carrier. The preferred buffering acid for use in these compositions is citric acid.

The present invention encompasses pharmaceutical compositions, containing tetracycline, which are used topically (as in the treatment of skin disorders, such as acne) without yellowing the skin of the user. The formulation of these compositions revolves around the discovery that by controlling the size and shape characteristics of the tetracycline compound as it recrystallizes on the skin after application, the tendency to yellow the skin can be eliminated. Generally, when applied to the skin from a solution, tetracycline recrystallizes at the skin surface in relatively large, yellow rhombohedron-shaped crystals, having an average size of at least about 35—40 µm. The flat surfaces of these crystals reflect light, imparting a yellow coloration to the skin. By formulating the compositions such that the tetracycline compound recrystallizes at the skin surface in small crystals, i.e., having an average size of 30 µm or less, preferably less than 25 µm, of irregular shape, the perception of skin yellowing is eliminated.

This result is achieved herein by controlling the pH of the composition. Thus, compositions of the present invention are those containing from 0.5% to 10%, preferably from 0.75% to 6%, most preferably from 1% to 3%, of an acid which buffers the compositions at a pH of from 2.0 to 4, preferably from 2.5 to 3.5. Examples of such acids include citric acid, lactic acid, ascorbic acid, glutaric acid, oxalic acid, hydrochloric acid, and mixtures thereof, with citric acid being preferred. In addition to the crystal modification effect, the lowering of the pH of the composition may act to shift its light absorption characteristics, thereby eliminating skin-yellowing.

Excellent therapeutic and skin yellowing results are also obtained where acid is added to the composition, bringing the pH down to from 2.0 to 2.5, and then an alkaline compound, such as sodium hydroxide, is added to bring the composition pH to from 3.0 to 3.5. Similar results are obtained where the composition is formulated to include a weak acid (e.g., citric acid) and its basic salts (e.g., sodium citrate), such that the final composition has a pH between 3.0 and 3.5 and contains from 0.75% to 6%, preferably from 1% to 3%, of the acid/salt mixture.

The compositions of the present invention contain from 0.005% to 5%, preferably from 0.05% to 2%, and most preferably about 0.5%, of tetracycline, a tetracycline salt or a tetracycline derivative. Mixtures of such tetracycline compounds may be used. The tetracycline compound contained in the composition must be present in solution; the skin yellowing effect is not present when the tetracycline compound is in a suspension, as in an ointment formulation. Tetracycline compounds useful in the present invention include tetracycline itself, as well as the salts thereof, for example, tetracycline hydrochloride and the tetracycline phosphate complex marketed under the trademarks "Tetrex" and "Panmycin Phosphate". Tetracycline analogues, for example, the oxytetracyclines or terramycins, such as terramycin hydrochloride and terramycin disodium salt dihydrate are also suitable herein. Preferred tetracycline compounds for use herein include tetracycline, tetracycline hydrochloride, and equilibrium mixtures of epi-tetracycline hydrochloride and tetracycline hydrochloride (formed on dissolution of tetracycline hydrochloride in water). Especially preferred tetracycline compounds include tetracycline hydrochloride and the equilibrium mixture of tetracycline hydrochloride and epi-tetracycline hydrochloride which forms spontaneously after aqueous solutions of tetracycline hydrochloride have been allowed to age at about 32.2°C (90°F) for about 7 days. In general, this equilibrium mixture comprises 40—45% by weight tetracycline hydrochloride and 55—60% by weight epi-tetracycline hydrochloride.

Other additives conventionally used in topical pharmaceutical compositions, such as perfumes, colorants, thickeners, preservatives, and penetration enhancers, may be included in the compositions of the present invention. Examples of such penetration enhancers include sugar esters, sulfoxides or phosphine oxides, and the combinations of these components described in US—A—3,896,238 and US—A—3,952,099. Compositions of the present invention may contain up to 10%, preferably from 0.1% to 1% by weight of a sugar ester of the type hereinafter disclosed. The sugar esters suitable for use in this invention can be classified as hydrocarbyl and alkyl polyoxyalkylene esters of cyclic polyhydroxy saccharides wherein one or more of the hydroxyl groups on the saccharide moiety is substituted with an acyl or polyoxyalkylene group.

Preferred sugar esters herein are those prepared by the esterification of sugars in a mole ratio of esterification agent:sugar of 1:1 and 2:1, i.e., the mono-acyl and di-acyl sugar esters. Especially preferred are the mono-acyl and di-acyl sugar esters wherein the acyl substituents contain from 8 to 20 carbon atoms. Of the mono-acyl and di-acyl sugar esters, the respective esters of di-saccharide sugars, especially sucrose, wherein the acyl groups contain from 8 to 20 carbon atoms are especially preferred. Sucrose monooleate has been found to be particularly efficacious.

By way of example, the following is a list of typical sugar esters suitable for use in the instant invention: glucose monooctanoate, glucose mono-caprate, glucose monolaurate, glucose monomyristate, galactose monopalmitate, galactose monostearate, galactose monooleate, mannose dipalmitate, xylose diooleate, xylose di-eicosanate, sucrose monooctanoate, sucrose monocaprate, sucrose monolaurate, sucrose monomyristate, sucrose monopalmitate, sucrose monostearate, sucrose monooleate, sucrose di-octanoate, sucrose dilaurate, sucrose dimyristate, sucrose dioleate, sucrose trilaurate, sucrose tripalmitate, sucrose trioleate, maltose monooleate, maltose dilaurate, galactose monolaurate, galactose dilaurate, cellibiose trilaurate, raffinose monomyristate, gentianose dioctanoate, and gentianose trioleate. Preferred sugar esters include sucrose monooctanoate, sucrose monodecanoate, sucrose monolaurate, sucrose monomyristate, sucrose monopalmitate, sucrose monostearate, sucrose monooleate, and sucrose dioleate.

Preferred compositions herein, having improved skin penetration properties, contain a compound selected from sulfoxides of the formula $R_1S(O)R_2$, wherein $R_1$ is a straight chain or branched chain alkyl, alkenyl, substituted alkyl, heteroalkyl or hydroxy-substituted alkyl substituent containing from 4 to 12 carbon atoms, and $R_2$ is a low molecular weight ($C_1$—$C_8$) alkyl or low molecular weight ($C_1$—$C_8$) hydroxy-substituted alkyl substituent; preferably $R_1$ is $C_{8-12}$ alkyl or hydroxyalkyl substituent and $R_2$ is $C_{1-4}$ alkyl or hydroxy-substituted alkyl substituent; and phosphine oxides of the formula $R_3R_4R_5P(O)$, wherein $R_3$ is an alkyl, aralkyl, substituted alkyl, heteroalkyl, or hydroxy-substituted alkyl substituent containing from 1 to 12 carbon atoms, or aryl (e.g., phenyl or tolyl) containing from 6 to 9 carbon atoms; and $R_4$ and $R_5$ are each low molecular weight alkyl ($C_1$—$C_4$) or low molecular weight hydroxy-substituted alkyl ($C_1$—$C_4$) substituents. Preferred herein are the alkyl sulfoxides wherein $R_1$ is an alkyl or hydroxyalkyl substituent containing from 8 to 12 carbon atoms and $R_2$ is a low molecular weight alkyl or low molecular weight hydroxyalkyl group containing from 1 to 8 carbon atoms. Alkyl tertiary phosphine oxides wherein $R_3$ is an alkyl or hydroxyalkyl substituent containing from 8 to 12 carbon atoms and $R_4$ and $R_5$ are each lower alkyl or lower hydroxyalkyl substituents are the preferred phosphine oxides herein.

The concentration of sulfoxide or phosphine component oxide employed in the preferred compositions of the present invention is at least 0.1% by weight of the composition and can range from 0.1% to 10% by weight. If concentrations less than 0.1% are used, the degree of penetration enhancement attained, especially with the lower chain lengths is not appreciable. If concentrations greater than 10% are employed, solubility problems may be encountered with the higher chain length materials. Preferably, the concentration of sulfoxide or phosphine oxide will range from 0.1% to 1% by weight of the total composition.

The balance of the compositions of the present invention, generally from 50% to 99.5% of the compositions, comprises a conventional pharmaceutical carrier material, generally in liquid or semi-liquid state, especially adapted for topical application. It is desirable that the carrier selected be capable of co-dissolving the materials used in the composition. Carrier materials suitable for use in the instant compositions include those well-known for use in the cosmetic and medical arts as bases for ointments, lotions, salves, aerosols, suppositories and the like. Suitable carriers include, for example, water, liquid alcohols, liquid glycols, liquid polyalkylene glycols, liquid esters, liquid amides, liquid protein hydrosylates, liquid alkylated protein hydrosylates, liquid lanolin and lanolin derivatives, and like materials commonly employed in cosmetic and medicinal compositions. Exemplary carriers include alcohols, including both monohydric and polyhydric alcohols, for example, ethanol, isopropanol, glycerol, sorbitol, 2-methoxyethanol, diethylene glycol, ethylene glycol, hexylene glycol, mannitol, and propylene glycol; ethers, such as diethyl or dipropyl ether; polyethylene glycols and methoxypolyoxyethylenes (such as carbowaxes having molecular weights ranging from 200 to 20,000); polyoxyethylene glycerols, polyoxyethylene sorbitols, and stearoyl diacetin. Oil-in-water emulsions such as cold cream bases, can also be used.

Preferably the carrier herein is a pharmaceutically-acceptable liquid alcohol containing from 2 to 6 carbon atoms. Mixtures comprising from 0 to 80% by weight of water and from 20 to 100% by weight of said $C_2$—$C_6$ alcohols are also suitable. Alcohols useful herein include ethanol, isopropanol and hexanol. Especially preferred carriers are water-ethanol mixtures at a weight ratio of from 1:20 to 5:1. Ethanol containing from 5 to 50% by weight of water, especially 40:60 by volume ethanol-water, is preferred as the carrier.

Because of storage stability considerations relating to tetracycline hydrochloride and epi-tetracycline hydrochloride when formulated in solution, for particular formulations it may be preferable to package the compositions of the present invention in kit form. In such cases, the tetracycline compound is packaged separately from any water-based carrier material, such as a mixture of ethyl alcohol, water, sucrose monooleate and decyl methyl sulfoxide, used in the composition. Accordingly, a preferred kit encompasses a separately packaged portion of the tetracycline component, such as an equilibrium mixture of tetracycline hydrochloride and epi-tetracycline hydrochloride and a separately packaged portion of a penetration enhancing carrier. Such separately packaged portions are stable on prolonged storage and can be admixed by the user immediately before the course of treatment. It is convenient to package the two portions to provide a sufficient quantity of the composition to last for about 4 to 8 weeks of treatment. This, of course, is not critical to the present invention inasmuch as the clinical efficacy of the compositions herein is satisfactory even after storage. However, the use of separate packages for the tetracycline active and for the fluid ointment base insures that fresh material will be provided to the user. The buffering acid component of the present invention, preferably citric acid, may be included either with the tetracycline portion or the liquid carrier portion of such a kit.

The size of the kits herein are of no consequence to the practice of the invention. For example, such kits can be provided which contain only a few grams of material and which are suitable for but a single application. Alternatively, kits can be provided which comprise a relatively large volume of the composition. The user can then measure aliquots of the carrier material and add thereto a premeasured packet of the tetracycline component. A preferred kit contains a separately packaged, fluid ointment base comprising from 0.1 to 1% by weight of sucrose monooleate, from 0.1 to 8% by weight of decyl methyl sulfoxide, from 30 to 70% by weight of water, from 30 to 70% by weight of ethyl alcohol, and from 1 to 6% by weight of citric acid. The second component of the kit comprises a separately packaged, dry portion of an anti-acne agent comprising an equilibrium mixture of tetracycline hydrochloride and epi-tetracycline hydrochloride in an amount sufficient to provide a 0.1% to 1% by weight concentration of said equilibrium mixture when dissolved in the fluid ointment base composition. The equilibrium mixture of tetracycline hydrochloride and epi-tetracycline hydrochloride may also contain from 0.05 to 0.15% by weight (based on the total weight of all components) of a color stabilizer, especially sodium bisulfite. Alternatively, the second component can be an equivalent amount of either tetracycline hydrochloride or epi-tetracycline hydrochloride, since these materials are stable in the dry state, and may contain the citric acid component rather than having it in the liquid portion of the kit.

Topical treatment regimens comprise applying the compositions herein directly to the skin, e.g., at the situs of a dermatosis, such as acne. The rate of application and duration of treatment will, of course, depend on the severity of the condition, the response of the particular patient, and related factors within the sound medical judgment of an attending physician. In general, for the compositions of the present invention, application rates of from 0.01 ml/cm$^2$ to 25 ml/cm$^2$ per day are used. Application can be made once, or preferably several times, daily for periods of a week or more, and do not result in a yellow coloration of the skin at the site of application.

The following examples illustrate topical compositions prepared and used in the manner of this invention, but are not intended to be limiting thereof.

Example I
The skin yellowing effect of the compositions of the present invention, as compared with control compositions, was tested as follows using a control composition having the following formulation.

Control

| Tetracycline hydrochloride | 154 mg |
|---|---|
| 4-Epitetracycline hydrochloride | 196 mg |
| n-Decyl methyl sulfoxide | 87.5 mg |
| Sucrose monooleate | 87.5 mg |
| Sodium bisulfite | 70 mg |

Vehicle=70 ml of a 40% (by volume) ethanol/water mixture.

Compositions of the present invention were formulated by adding citric acid to the control composition in an amount such that the final composition contained 1%, by weight, of the citric acid (Composition A) and another composition was formed such that the final composition contained 2% citric acid (Composition B). Compositions A and B both have pH's between about 2.5 and about 3.5. These compositions were applied to skin once at 4 ml/cm². The skin yellowing effect of these compositions was then graded on a 0 to 5 grading scale, with 0 indicating the yellow staining effect of the control compositions and 5 indicating no yellow skin coloration at all. The tests were repeated on 8 subjects and the skin staining was evaluated by two graders for each subject. The results were averaged and are summarized in the table below.

| Test | Control | Composition A | Composition B |
|---|---|---|---|
| 1 | 0 | 4.25 | 4.75 |
| 2 | 0 | — | 4.40 |

The benefit in terms of minimization of skin yellowing is dramatically demonstrated by the data contained in the above table. Substantially similar results are obtained where the citric acid component is replaced, in whole or in part, by lactic acid, ascorbic acid, hydrochloric acid, glutaric acid, oxalic acid, or mixtures of these components. Substantially similar results are also obtained where a composition similar to Composition A is formulated but with the amount of citric acid being 0.5%, 0.75%, 1.5%, 3%, 4%, 5%, 7%, or 8% of the final composition. Similar results are also obtained where the tetracycline components in the above compositions are replaced, in whole or in part, by tetracycline, tetracycline phosphate complexes, terramycin hydrochloride, terramycin disodium salt dihydrate, or mixtures of these components.

A study of crystals formed on the skin surface in the above test indicates that the control composition resulted in the formation of crystals having a rhombohedral shape and an average size of greater than 30 μm, while the crystals formed from Composition B on the skin surface had an average crystal size of less than 28 μm and were irregular in shape.

Example II

The control composition, described in Example I, and compositions of the present invention, formulated by adding amounts of citric acid ranging from 0.5% to 10%, by weight, of the final composition to the control were applied to white press-apply labels; the amount applied was approximately 3 ml/cm². The color differences obtained from these compositions, as judged under white light, were found to be as follows:

(1) Composition obtained by adding 0.5% citric acid to the control resulted in noticeably less yellow color than did the control composition.

(2) Compositions formulated by adding 2 to 4% citric acid to the control composition resulted in noticeably less yellow coloration than that obtained using the control plus 0.5% citric acid and, when compared to the control, showed almost no yellow coloration.

(3) Levels of citric acid above the range of 2—4% did not give any significant improvement over the 2—4% citric acid level, but were significantly better than the control composition.

Substantially similar results are obtained where the citric acid is replaced, in whole or in part, by lactic acid, ascorbic acid, hydrochloric acid, glutaric acid, oxalic acid, and mixtures thereof.

Example III

A composition of the present invention, having the formulation given below, is formulated in a conventional manner.

| Component | Weight % |
|---|---|
| Tetracycline hydrochloride | 0.22 |
| 4-Epitetracycline hydrochloride | 0.28 |
| Citric acid | 2.0 |
| Sodium bisulfite | 0.1 |
| Propylene glycol monoisostearate | 1.5 |
| 40/60 (approximately) ethanol/water | Balance to 100 |

This composition when applied to the skin at an application rate of about 10 mg/cm² per day, provides effective therapy in the treatment of acne without causing the treated skin area to become yellow.

Example IV

A composition of the present invention, having the formulation given below, is formulated in a conventional manner.

| Component | Weight % |
|---|---|
| Tetracycline hydrochloride | 0.22 |
| 4-Epitetracycline hydrochloride | 0.22 |
| Citric acid | 2.0 |
| Sodium bisulfite | 0.1 |
| Myristyl alcohol | 1—5 |
| 40/60 (approximately) ethanol/water | Balance to 100 |

This composition, when applied in a daily dosage of about 5 ml/cm², provides an effective topical treatment for a variety of skin disorders, including acne, without causing the treated skin area to become yellow stained.

Example V

A composition of the present invention, having the formulation given below, is formulated in a conventional manner.

| Component | |
|---|---|
| Tetracycline hydrochloride | 2.2 mg/ml |
| 4-Epitetracycline hydrochloride | 3.25 mg/ml |
| Decyl methyl sulfoxide | 1.25 mg/ml |
| Citric acid | 18.95 mg/ml |
| Sodium bisulfite | 1 mg/ml |

Appropriate amounts of the above components are dissolved in 40/60 ethanol/water vehicle to form about 70 ml of the desired composition. The composition has a pH between about 2.5 and 3.0. This composition, when applied in a daily dosage of about 5 ml/cm², provides an effective topical treatment for a variety of skin disorders, including acne, without causing the treated area to become yellow stained. Excellent results are also obtained where sodium hydroxide is added to the above composition in an amount to raise the pH of the composition to 3.5.

Example VI

A composition, effective in the topical treatment of acne, sold in kit form, and having the formulation given below is made in a conventional manner.

| Component | Weight |
|---|---|
| **Component 1** | |
| Tetracycline hydrochloride/epi-tetracycline hydrochloride mixture | 1.70 g (0.06 oz) |
| Sodium bisulfite | 0.57 g (0.02 oz) |
| Citric acid | 7.09 g (0.25 oz) |
| **Component 2** | |
| Ethanol | 113.4 g (4.0 oz) |
| Sucrose monooleate | 1.70 g (0.06 oz) |
| Decyl methyl sulfoxide | 1.70 g (0.06 oz) |
| Water | 70.88 g (2.5 oz) |

6

In the above, Component 1 is packaged in a dry, water-proof foil packet; Component 2 is packaged in a bottle having sufficient head space to allow mixing. Immediately prior to use, Component 1 is added to Component 2 and the mixture is shaken to mix. The user applies an effective amount of the composition to the acne lesions, ad lib, and alleviation of the acne is secured; such use does not cause the treated situs to take on a yellow coloration.

In the above, the citric acid component may be included in Component 2 rather than in Component 1, while retaining its yellow stain reduction benefit. Further, in the above example, the mixture of tetracycline hydrochloride and epi-tetracycline hydrochloride may be replaced by tetracycline hydrochloride and epi-tetracycline hydrochloride, respectively, and equivalent results are secured. Similar results are also obtained where the above example is formulated without the sucrose monooleate and/or the sodium bisulfite components.

## Claims

1. A topical composition, for use in the treatment of skin disorders, comprising from 0.005% to 5% of a tetracycline compound selected from tetracycline, a tetracycline salt or a tetracycline derivative in solution together with a pharmaceutically-acceptable topical carrier, characterized in that said composition contains from 0.5% to 10% of a pharmaceutically-acceptable acid which buffers the composition at a pH of from 2.0 to 4.0.

2. A composition according to Claim 1 characterized in that said pharmaceutically-acceptable acid is selected from citric acid, lactic acid, ascorbic acid, hydrochloric acid, glutaric acid, oxalic acid, and mixtures thereof.

3. A composition according to Claims 1 or 2 characterized in that said pharmaceutically-acceptable acid is citric acid.

4. A composition according to any one of Claims 1 to 3 characterized in that said composition contains from 1% to 3% of the pharmaceutically-acceptable acid.

5. A composition according to any one of Claims 1 to 4 characterized in that said composition is buffered at a pH of from 2.5 to 3.5.

6. A composition according to any one of Claims 1 to 5 characterized in that said composition additionally contains up to 10% of a sugar ester and from 0.1% to 10% of a sulfoxide having the formula $R_1S(O)R_2$ wherein $R_1$ is a $C_8$—$C_{12}$ alkyl or hydroxyalkyl substituent and $R_2$ is a substituent selected from $C_1$—$C_4$ alkyl and hydroxy-substituted alkyl substituents.

7. A composition according to any one of Claims 1 to 6 characterized in that said composition additionally contains an alkaline salt of the pharmaceutically-acceptable acid in an amount such that the composition contains from 0.75% to 6% of the acid and salt together, and has a pH of from 3.0 to 3.5.

## Patentansprüche

1. Äußerlich anwendbare Zubereitung zur Verwendung bei der Behandlung von Hautkrankheiten, umfassend 0.005 bis 5% einer Tetracyclinverbindung ausgewählt aus Tetracyclin, einem Tetracyclinsalz oder einem Tetracyclinderivat in Lösung zusammen mit einem pharmazeutisch verträglichen äußerlich anwendbaren Träger, dadurch gekennzeichnet, daß die Zubereitung 0,5 bis 10% einer pharmazeutisch verträglichen Säure enthält, welche die Zubereitung auf einen pH-Wert von 2,0 bis 4,0 puffert.

2. Zubereitung gemäß Anspruch 1, dadurch gekennzeichnet, daß die pharmazeutisch verträgliche Säure ausgewählt ist aus Zitronensäure, Milchsäure, Ascorbinsäure, Chlorwasserstoffsäure, Glutarsäure, Oxalsäure und Gemischen daraus.

3. Zubereitung gemäß Anspruch 1 oder 2, dadurch gekennzeichnet, daß die pharmazeutisch verträgliche Säure Zitronensäure ist.

4. Zubereitung gemäß einem der Ansprüche 1 bis 3, dadurch gekennzeichnet, daß die Zubereitung 1 bis 3% der pharmazeutisch verträglichen Säure enthält.

5. Zubereitung gemäß einem der Ansprüche 1 bis 4, dadurch gekennzeichnet, daß die Zubereitung auf einen pH-Wert von 2,5 bis 3,5 gepuffert ist.

6. Zubereitung gemäß einem der Ansprüche 1 bis 5, dadurch gekennzeichnet, daß die Zubereitung zusätzlich bis zu 10% eines Zuckeresters und 0,1 bis 10% eines Sulfoxids der Formel $R_1S(O)R_2$ enthält, worin $R_1$ ein $C_8$—$C_{12}$-Alkyl- oder -Hydroxyalkylsubstituent ist und $R_2$ ein Substituent ausgewählt aus $C_1$—$C_4$-Alkyl- und hydroxysubstituierten Alkylsubstituenten ist.

7. Zubereitung gemäß einem der Ansprüche 1 bis 6, dadurch gekennzeichnet, daß die Zubereitung zusätzlich eine basisches Salz der pharmazeutisch verträglichen Säure in einer solchen Menge enthält, daß die Zubereitung 0,75 bis 6% der Säure und des Salzes zusammengenommen enthält, und einen pH-Wert von 3,0 bis 3,5 aufweist.

## Revendications

1. Composition topique utilisable dans le traitement des affections de la peau, comprenant de 0,005% à 5% d'un composé de tétracycline choisi parmi la tétracycline, un sel de tétracycline ou un dérivé de tétracycline en solution avec un véhicule topique pharmaceutiquement acceptable, caractérisée en ce que ladite composition contient de 0,5% à 10% d'un acide pharmaceutiquement acceptable qui tamponne la composition à un pH de 2,0 à 4,0.

2. Composition selon la revendication 1, caractérisée en ce que ledit acide pharmaceutiquement acceptable est choisi parmi l'acide citrique, l'acide lactique, l'acide ascorbique, l'acide chlorhydrique, l'acide glutarique, l'acide oxalique, et leurs mélanges.

3. Composition selon la revendication 1 ou 2, caractérisée en ce que l'acide pharmaceutiquement acceptable est l'acide citrique.

4. Composition selon l'une quelconque des revendications 1 à 3, caractérisée en ce que ladite composition contient de 1% à 3% de l'acide pharmaceutiquement acceptable.

5. Composition selon l'une quelconque des revendications 1 à 4, caractérisée en ce que ladite composition est tamponnée à un pH de 2,5 à 3,5.

6. Composition selon l'une quelconque des revendications 1 à 5, caractérisée en ce que ladite composition contient en plus jusqu'à 10% d'un ester de sucre et de 0,1% à 10% d'un sulfoxyde répondant à la formule $R_1S(O)R_2$, dans laquelle $R_1$ est un substituant alkyle ou hydroxyalkyle en $C_8$—$C_{12}$ et $R_2$ est un substituant choisi parmi les substituants alkyle et hydroxyalkyle en $C_1$—$C_4$.

7. Composition selon l'une quelconque des revendications 1 à 6, caractérisée en ce que ladite composition contient en plus un sel alcalin de l'acide pharmaceutiquement acceptable, en une quantité telle que la composition contient de 0,75% à 6% de l'acide et du sel ensemble et à un pH de 3,0 à 3,5.